# EUROPEAN PATENT SPECIFICATION

(11) **EP 1 019 149 B1**
(45) Date of publication and mention of the grant of the patent: **22.09.2004**
(21) Application number: 98951027.6
(22) Date of filing: 09.10.1998
(51) Int. Cl.: A61N 7/00

(54) **ULTRASONIC DELIVERY SYSTEM**
ULTRASCHALLABGABEVORRICHTUNG
SYSTEME D'EMISSION ULTRASONORE

(30) Priority: 09.10.1997 US 61630 P
(43) Date of publication of application: 19.07.2000
(73) Proprietor: Exogen, Inc., Memphis, Tennessee 38116 (US)
(72) Inventor: URGOVITCH, Kenneth, J., Sr., Clifton, NJ 07013 (US); KROMPASICK, Donald, E., Bethlehem, PA 18020 (US); TALISH, Roger, J., Hillsborough, NJ 08876 (US)
(74) Representative: Laufhütte, Dieter, Dr.-Ing.
(86) International application number: PCT/US1998/021366
(87) International publication number: WO 1999/019025

(56) References cited:
- EP-A- 0 695 559
- WO-A-97/33649

## Description

### BACKGROUND OF THE INVENTION

### 1. Field of the Invention

This disclosure relates to the generation and delivery of ultrasound signals and, more particularly, to an ultrasonic delivery system for use with at least one ultrasonic transducer to accelerate the process of healing in both hard and soft tissue.

### 2. Description of the Related Art

The therapeutic value of ultrasonic waves is known. Various techniques and devices are used to apply ultrasound waves to various areas of the body. In one known technique a pulsed radio-frequency ultrasonic signal applied via a transducer to the skin of a patient is directed to the site of the wound. The radio-frequency signal is in the range of 1.3 to 2 MHz, and it consists of pulses at a repetition rate of 100 to 1000 Hz, with each pulse having a duration in the range of 10 to 20,000 microseconds. See, e.g. U.S. Patent No. 4,530,360 to Duarte.

U.S. Patent Nos. 5,003,965 and 5,186,162 both to Talish and Lifshey ("Talish '965" and "Talish '162", respectively) describe an ultrasonic delivery system where the R-F generator and transducer are both part of a modular applicator unit that is placed at the skin location. The signals controlling the duration of ultrasonic pulses and the pulse repetition frequency are generated apart from the applicator unit. Talish '965 and Talish ' 162 also describe fixture apparatus for attaching the applicator unit so that the operative surface is adjacent the skin location. In Talish '965 and Talish' 162, the skin is surrounded by a cast, while in U.S. Patent No. 5,211,160 to Talish and Lifshey ("Talish '160") fixture apparatus is described for mounting on uncovered body parts (i.e., without a cast or other medical wrapping). Talish 160 also describes various improvements to the applicator unit.

Document WO 9733649 to Talish discloses an apparatus according to the preamble of claim 1.

As ultrasonic self-treatment becomes more popular, a need arises to make ultrasonic delivery systems easier and more convenient to use. Patients who suffer from back injuries are inconvenienced by body casts or braces that limit motion of the spinal column for extended periods of time, often months. Where, for example, the patient has to undergo surgery to fuse several vertebrae, a back brace is required to limit the movement of the patient's torso until the bones can grow together. Ultrasonic treatment can be used to accelerate the healing process and reduce the amount of time the patient is required to wear a cast or brace.

If ultrasonic treatment is employed, the back support brace must be removed in order to administer treatment. It is not preferred to remove the back support which is employed to maintain the patient's spine in a fixed position. A need exists to be able to administer ultrasonic treatment without moving the spinal support. It is also desirable to be able to provide ultrasonic treatment to multiple regions of the patient' body simultaneously, where multiple injuries have been sustained by the patient.

### SUMMARY OF THE INVENTION

An ultrasonic delivery system includes a brace member adjustably configurable to provide support to a human spinal column. The brace defines a cavity suitable for being disposed adjacent to a predetermined location overlying the human spinal column. An ultrasound generating assembly includes at least one treatment head module dimensioned to be positioned in the cavity of the brace member, and a main operating unit provides excitation signals to the at least one treatment head module.

### BRIEF DESCRIPTION OF DRAWINGS

The invention will be described in detail in the following description of preferred embodiments with reference to the following figures wherein:
FIG. 1 is an exploded isometric view of an ultrasonic delivery system;
FIG. 2 is an exploded isometric view showing more detail for a back member;
FIG. 3 is an exploded isometric view showing more detail for a front member;
FIG. 4 is a front view of a patient using an embodiment of an ultrasonic delivery system;
FIG. 5 is a side view of a patient using an embodiment of an ultrasonic delivery system;
FIG. 6 shows a cross-sectional view as indicated in FIG. 5;
FIG. 7 shows an alternate embodiment of an ultrasonic delivery system being used by a patient;
FIG. 8 shows a cross-sectional view as indicated in FIG. 7; and
FIG. 9 is an exploded isometric view of a back member of alternate preferred embodiment.

### DETAILED DESCRIPTION OF PREFERRED EMBODIMENTS

The present disclosure describes the use of an ultrasonic treatment apparatus for treating spinal injuries. At least one ultrasonic treatment head module is received within a brace member which provides support to the patients spinal column. The brace member does not have to be removed in order to administer ultrasonic treatment. Further, the brace member provides locations for maintaining multiple transducer head modules in a predetermined location whereby ultrasonic radiation is propagated into the injured areas of the patient.

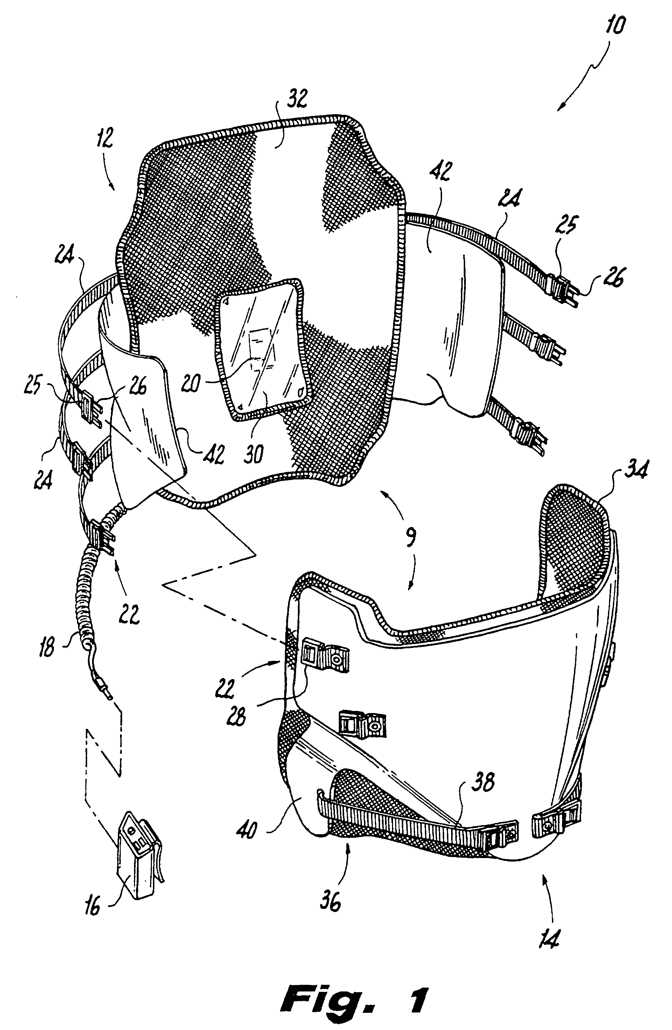

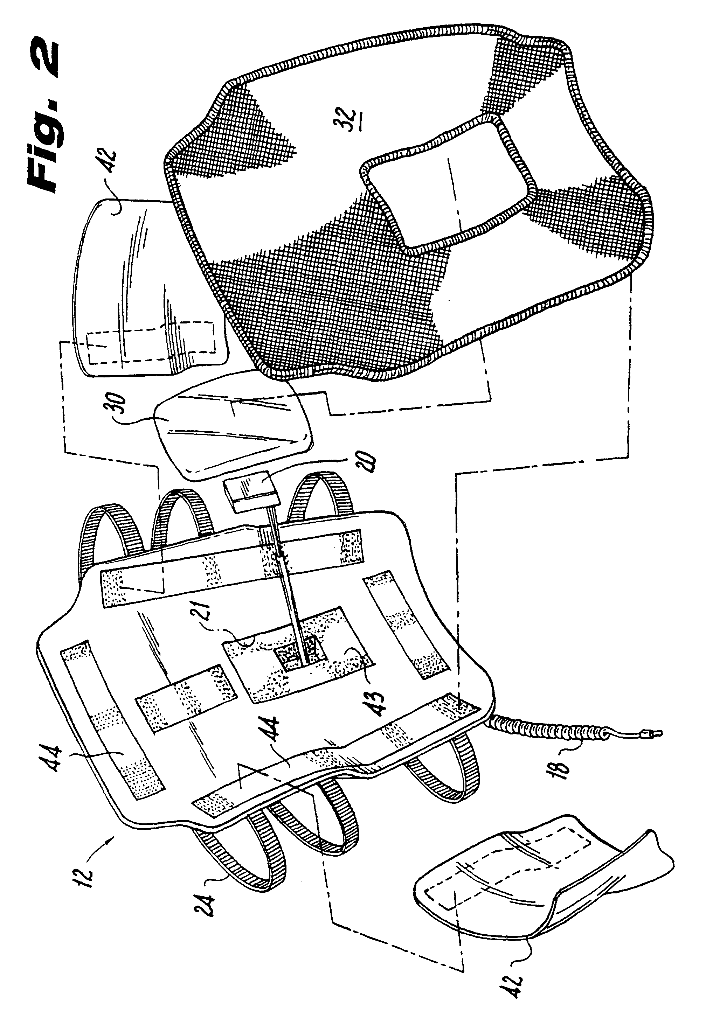

Referring now in specific detail to the drawings in which like reference numerals identify similar or identical elements throughout the several views, and initially to FIG. 1, one embodiment of an ultrasonic delivery system constructed in accordance with the present disclosure is shown generally as assembly 10. Assembly 10 includes a rigid brace member 9 including a back member 12 and a front member 14 which are positioned about a patient and adjustably configured to create an appropriate fit. Brace member 9 is used for supporting the spinal column of a patient. Back member 12 has a recessed portion 21 which defines a cavity for receiving a support pad 43 which mounts treatment head module 20 (see FIG. 2). Transducer head module is connected to an interface cable 18 which is connected to a main operating unit 16. Main operating unit 16 supplies the control signals and power to operate treatment head module 20. Treatment head module 20 may have a pad 30 mounted on it or have a pad 30 independent of treatment head module. Pad 30 contains ultrasonic conductive material, preferably conductive gel for propagating ultrasound waves into the patient.

Back member 12 is adjustably configured with respect to front member 14 by a securing means 22. Securing means 22 may include a plurality of belts 24 mounted on back member 12. Each belt 24 has a snap 26 which is received in a corresponding receptacle 28 located on rigid front member 14. Belt 24 is adjusted by a buckle 25.

Back member 12 and front member 14 are lined by a padded back insert 32 and a padded front insert 34, respectively. Padded back insert 32 and padded front insert 34 add to the comfort of the patient when ultrasonic delivery system 10 is in use. Padded back insert 32 has an opening formed therethrough to allow pad 30 to contact the patients skin directly in the area of treatment. Padded back insert 32 and padded front insert 34 may be attached to the inside of back member 12 and front member 14, respectively using hook and loop pads 44 or other means known in the art.

Referring to FIG. 2, back support 12 is shown in greater detail. Back support 12 is contoured to the patient's body. Further, detachable side members 42 are installed to back member 12 to allow a better and more secure fit for the patient. Side members 42 are adjustable to allow ultrasonic deliver system 10 to be used by different sized patients. In one embodiment, side members 42 can be attached and adjusted using hook and loop pads 44. Recessed portion 21 may be configured with a support pad 43. Support pad 43 defines an opening for receiving treatment head module 20. The opening in support pad 43 is configured to allow adjustment of the location of treatment head module 20 over an injured area. The opening in padded back insert 32, the cavity defined in recessed portion 21 and pad 30 may all be dimensioned to allow for treatment head module 20 placement to be adjustable over a large area of the patient's body.

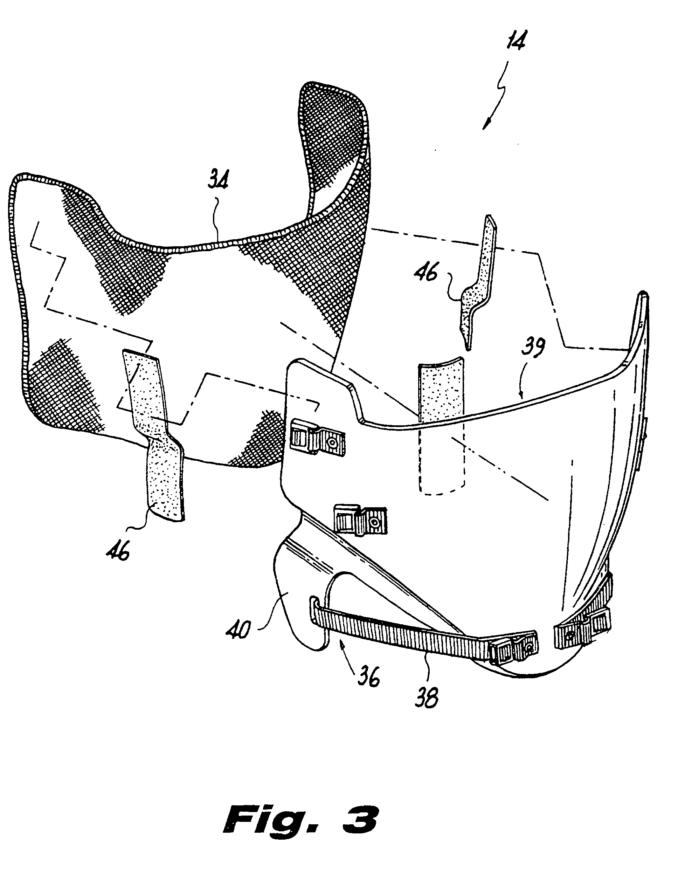

Referring now to FIG. 3, front member 14 has an adjustment means 36 to configure the brace member 9 to a patient. Adjustment means 36 includes extended portions 40 located laterally on opposite sides of front member 14. Extended portions 40 have a hole therethrough for receiving an adjustable strap 38 for tightening and drawing the extended portions 40 inward toward the patient. To provide further comfort for patients, reliefs 46 are supplied. Reliefs 46 prevent front member 14 from causing irritation of the patients abdomen due to an upper edge 39 of front member 14. Reliefs 46 are placed between front member 14 and padded front insert 34 at upper edge 39. If the patient leans forward while brace member 9 is in use, reliefs 46 engage the patient's abdomen first and act as a lever moving upper edge 39 away from the patient and hence protecting the patient from discomfort.

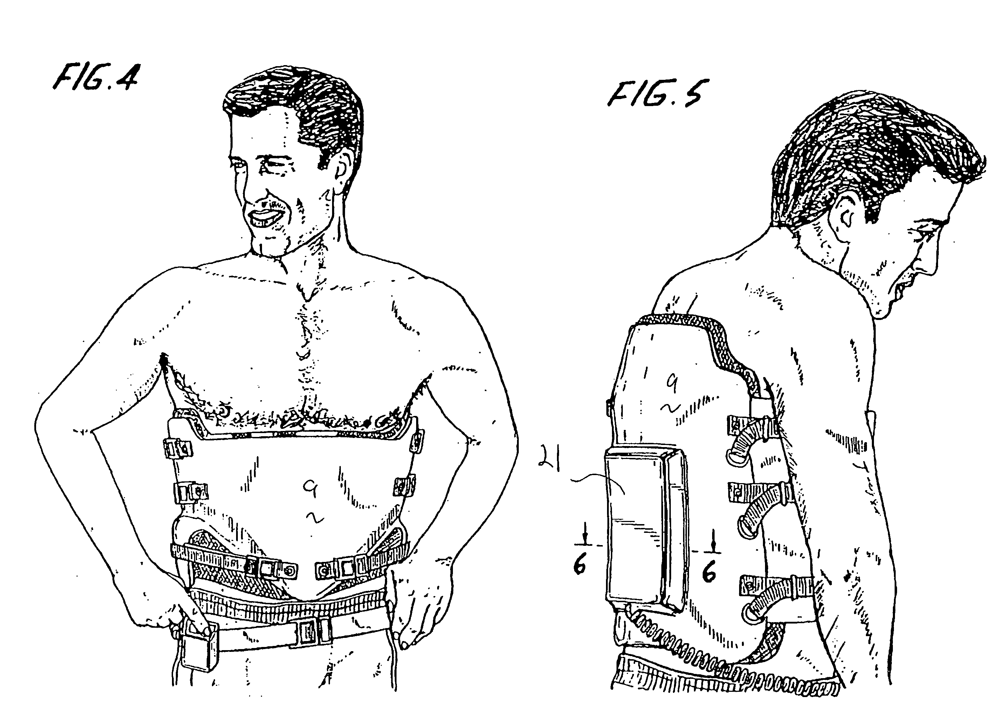

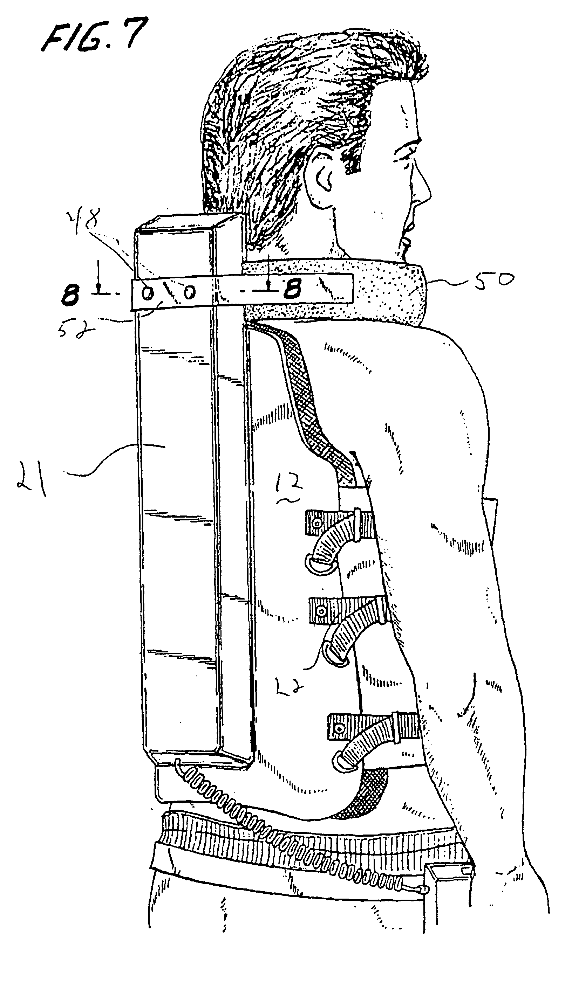

Referring to FIGS. 4, 5 and 6, a patient is shown wearing brace member 9. FIGS. 4 and 5 illustrate the fit of brace member 9 on a patient. FIG. 6 shows a cross section of ultrasonic delivery system 10 in use. Recessed portion 21 of back member 12 receives support pad 43 which supports treatment head module 20. Treatment head module 20 propagates ultrasound waves into pad 30 which pass through skin 62 into spinal column 58 for healing vertebral body 60. Padded back insert 32 is shown defining an opening for pad 30.

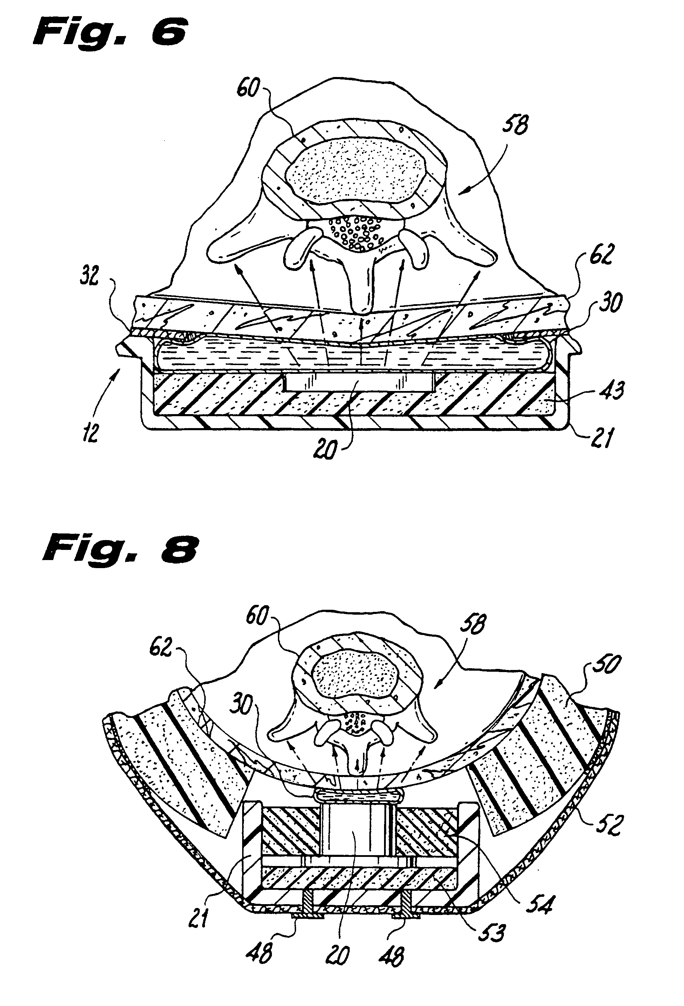

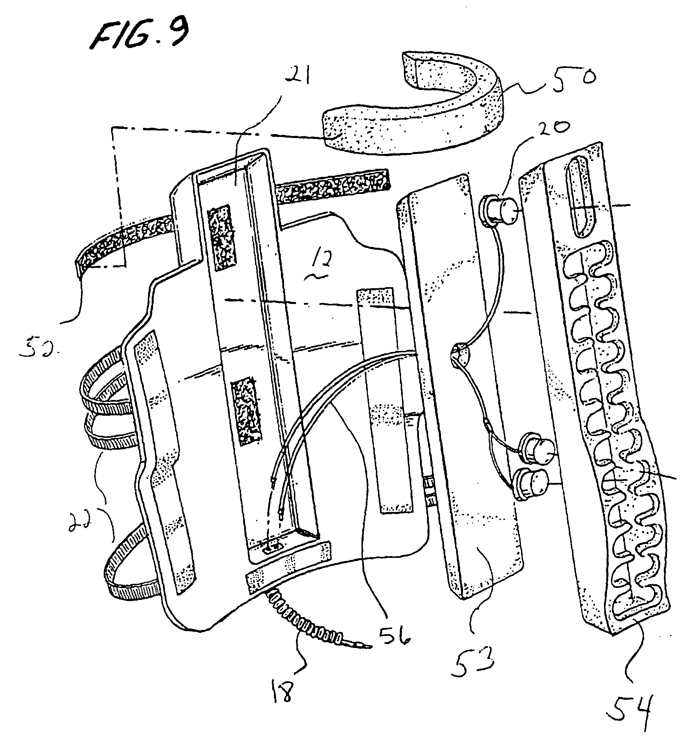

Referring now to FIGS. 7, 8 and 9 an alternate embodiment of ultrasonic delivery system 10 includes a configuration for treatment of the entire spinal column or a part thereof. FIG. 8 illustrates rigid back member 12 with recessed portion 21 extending the length of the patient's spinal column and forming a cavity. Further details are presented in FIG. 9. Recessed portion 21 adjacent to the patient's neck has a neck brace support 52 to secure neck brace 50 which can be attached with screws 48. Neck brace 50 and neck brace support 52 work in conjunction with securing means 22 to secure the patient's spinal column to prevent motion thereof. Backing pad 53 occupies vacant space within recessed area 21. Backing pad 43 defines an opening for the passage of cables 56, and further provides support for treatment head modules 20 to prevent them from backing away from the patients skin. A treatment head receptacle 54 holds a plurality of treatment head modules 20 adjacent to the patients spinal column. Treatment head receptacle 54 allows a doctor or technician to vertically or horizontally adjust treatment head modules 20 to locate them in appropriate positions to provide maximum effectiveness in accelerating the healing process.

In a preferred embodiment, treatment head receptacle 54 is formed from a compressible material, for example, styrene foam. Treatment head receptacle 54 is compressed against backing pad 53 and treatment head modules 20 are pressed against the patient's skin. It is also contemplated that treatment head modules 20 have pads 30 installed thereon to propagate ultrasound waves into the patient. Pads 30 contain an ultrasound conducting material preferably conductive gel. Each treatment head 20 is placed over a different portion of the spinal column. Each treatment module can be activated with varying control signals. Parameters that can be controlled include durations of operation, frequencies of operation and/or power levels as required for individual patient treatment.

Referring to FIG. 8, a cross-section at the patients neck shows ultrasonic delivery system 10 during operation. Neck brace support 52 is shown mounted to recessed portion 21 of back member 12. Neck brace support may be attached to neck brace 52 by hook and loop or other attachment means known in the art. Backing pad 53 is secured within recessed portion 21 and supports treatment head module 20. Treatment head module 20 is also secured by treatment head receptacle 54. Treatment head module 20 is connected to pad 30 which is in contact with skin 62. Ultrasonic waves propagate from treatment head module 20 through pad 30 and skin 62 reaching spinal column 58 and accelerating healing in vertebral body 60.

## Claims

1. An ultrasonic delivery system comprising
a brace member (9) adjustably configurable to provide support to a human spinal column, said brace member defining a cavity (21) suitable for being disposed adjacent to a predetermined location overlying the human spinal column and comprising a back member (12), and
an ultrasound generating assembly including at least one treatment head module (20) dimensioned to be positioned in the cavity (21) of the brace member and a main operating unit (16) for providing excitation signals to the at least one treatment head module, **characterised in that** said brace member is rigid and comprises a rigig front member (14).

2. An ultrasonic delivery system as recited in claim 1, wherein the brace member (9) further comprises a padded insert (32, 34).

3. An ultrasonic delivery system as recited in claim 1, wherein the brace member further comprises a treatment head receptacle for adjustably positioning the at least one treatment head module (20).

4. An ultrasonic delivery system as recited in claim 1, further comprising a pad (30) for conducting ultrasound waves disposed on the at least one treatment head module.

5. An ultrasonic delivery system as recited in claim 3, wherein said treatment head receptacle is disposed within said cavity (21), said treatment head module is dimensioned to be adjustably positioned within said treatment head receptacle, and a pad (30) is disposed on the at least one treatment head module.

6. An ultrasonic delivery system as recited in claim 5, wherein said back member (12) defines said cavity (21), a pair of side members (42) is provided adjustably mounted to either the front member (14) or back member (12), a padded front insert (34) and padded back insert (32) are coupled to the front member (14) and back member (12), respectively, the padded back member (12) forming an opening, and said pad comprises a gel pad (30) passing through the opening in said padded back insert (32).

7. An ultrasonic delivery system as recited in one of the claims 1 to 6, wherein the at least one treatment head module is a plurality of treatment head modules which are adapted to receive varying control signals.

## Patentansprüche

1. Ein Ultraschallabgabesystem, das Folgendes beinhaltet:
ein Korsettelement (9), das einstellbar konfigurierbar ist, um einer menschlichen Wirbelsäule Halt zu geben, wobei das Korsettelement einen Hohlraum (21) definiert, der zum Anordnen anliegend an eine vorbestimmte Stelle, die über der menschlichen Wirbelsäule liegt, geeignet ist, und ein Rückenelement (12) beinhaltet, und
eine Ultraschallerzeugungsanordung, die mindestens ein Behandlungskopfmodul (20) umfasst, das dimensioniert ist, um in dem Hohlraum (21) des Korsettelements positioniert zu werden, und eine Hauptbedieneinheit (16), um dem mindestens einen Behandlungskopfmodul Anregungssignale bereitzustellen, **dadurch gekennzeichnet, dass** das Korsettelement unbiegsam ist und ein unbiegsames Vorderelement (14) beinhaltet.

2. Ultraschallabgabesystem gemäß Anspruch 1, wobei das Korsettelement (9) ferner einen gepolsterten Einsatz (32, 34) beinhaltet.

3. Ultraschallabgabesystem gemäß Anspruch 1, wobei das Korsettelement ferner ein Behandlungskopfbehältnis für die einstellbare Positionierung des mindestens einen Behandlungskopfmoduls (20) beinhaltet.

4. Ultraschallabgabesystem gemäß Anspruch 1, das ferner ein Polster (30) zum Leiten von Ultraschallwellen, die auf dem mindestens einen Behandlungskopfmodul angeordnet sind, beinhaltet.

5. Ultraschallabgabesystem gemäß Anspruch 3, wobei das Behandlungskopfbehältnis innerhalb des Hohlraums (21) angeordnet ist, wobei das Behandlungskopfmodul dimensioniert ist, um einstellbar innerhalb des Behandlungskopfbehältnisses positioniert zu werden, und ein Polster (30) auf dem mindestens einen Behandlungskopfmodul angeordnet ist.

6. Ultraschallabgabesystem gemäß Anspruch 5, wobei das Rückenelement (12) den Hohlraum (21) definiert, ein Paar Seitenelemente (42) einstellbar auf entweder dem Vorderelement (14) oder dem Rückenelement (12) montiert ist, ein gepolsterter Vordereinsatz (34) und ein gepolsterter Rückeneinsatz (32) jeweils mit dem Vorderelement (14) und dem Rückenelement (12) gekoppelt sind, wobei das gepolsterte Rückenelement (12) eine Öffung bildet, und das Polster ein Gel-Polster (30) beinhaltet, das durch die Öffnung in dem gepolsterten Rückeneinsatz (32) verläuft.

7. Ultraschallabgabesystem gemäß einem der Ansprüche 1 bis 6, wobei das mindestens eine Behandlungskopfmodul eine Vielzahl von Behandlungskopfmodulen ist, die angepasst sind, um variierende Steuersignale zu empfangen.

## Revendications

1. Un système d'administration à ultrasons comportant
un élément formant armature (9) pouvant être configuré de façon à être ajustable pour fournir un soutien à une colonne vertébrale humaine, ledit élément formant armature définissant une cavité (21) adaptée pour être disposée adjacente à un endroit prédéterminé recouvrant la colonne vertébrale humaine et comportant un élément de derrière (12), et
un assemblage générant des ultrasons comprenant au moins un module de tête de traitement (20) dimensionné pour être positionné dans la cavité (21) de l'élément formant armature et une unité de commande principale (16) destinée à fournir des signaux d'excitation à cet au moins un module de tête de traitement, **caractérisé en ce que** ledit élément formant armature est rigide et comporte un élément de devant rigide (14).

2. Un système d'administration à ultrasons tel qu'énoncé dans la revendication 1, dans lequel l'élément formant armature (9) comporte en outre une pièce d'insertion rembourrée (32, 34).

3. Un système d'administration à ultrasons tel qu'énoncé dans la revendication 1, dans lequel l'élément formant armature comporte en outre un réceptacle de tête de traitement destiné à positionner de façon ajustable cet au moins un module de tête de traitement (20).

4. Un système d'administration à ultrasons tel qu'énoncé dans la revendication 1, comportant en outre un tampon (30) destiné à conduire des ondes ultrasonores disposé sur cet au moins un module de tête de traitement.

5. Un système d'administration à ultrasons tel qu'énoncé dans la revendication 3, dans lequel ledit réceptacle de tête de traitement est disposé au sein de ladite cavité (21), ledit module de tête de traitement est dimensionné pour être positionné de façon ajustable au sein dudit réceptacle de tête de traitement, et un tampon (30) est disposé sur cet au moins un module de tête de traitement.

6. Un système d'administration à ultrasons tel qu'énoncé dans la revendication 5, dans lequel ledit élément de derrière (12) définit ladite cavité (21), une paire d'éléments latéraux (42) est fournie et montée de façon ajustable sur soit l'élément de devant (14), soit l'élément de derrière (12), une pièce d'insertion de devant rembourrée (34) et une pièce d'insertion de derrière rembourrée (32) sont couplées à l'élément de devant (14) et à l'élément de derrière (12), respectivement, l'élément de derrière rembourré (12) formant une ouverture, et ledit tampon comporte un tampon de gel (30) passant à travers l'ouverture dans ladite pièce d'insertion de derrière rembourrée (32).

7. Un système d'administration à ultrasons tel qu'énoncé dans l'une des revendications 1 à 6, dans lequel cet au moins un module de tête de traitement est une pluralité de modules de têtes de traitement qui sont adaptés pour recevoir des signaux de commande variables.
